Europäisches Patentamt
European Patent Office
Office européen des brevets

(19) (11) Veröffentlichungsnummer: **0 203 441 A2**

(12) # EUROPÄISCHE PATENTANMELDUNG

(21) Anmeldenummer: **86106401.2**

(22) Anmeldetag: **12.05.86**

(51) Int. Cl.⁴: **C 07 D 455/06**, A 61 K 31/435, C 07 D 491/14 // (C07D491/14, 317:00, 221:00, 221:00)

(30) Priorität: **25.05.85 DE 3518917**

(43) Veröffentlichungstag der Anmeldung: **03.12.86 Patentblatt 86/49**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Stegelmeier, Hartmut, Dr., Meide 1d, D-4010 Hilden (DE)**
Erfinder: **Ingendoh, Axel, Dr., Unterste Dillenberg 18, D-5620 Velbert 15 (DE)**
Erfinder: **Muisers, Hans-Ferdinand, Dr., Hahnenweg 5, D-5000 Köln 60 (DE)**
Erfinder: **Lange, Walter, Dr., Neusser Strasse 321, D-5000 Köln 60 (DE)**
Erfinder: **Kazda, Stanislav, Dr., Gellertweg 18, D-5600 Wuppertal 1 (DE)**
Erfinder: **Knorr, Andreas, Dr., Pahlkestrasse 15, D-5600 Wuppertal 1 (DE)**
Erfinder: **Garthoff, Bernward, Dr., Händelstrasse 22, D-4010 Hilden (DE)**

(54) **Substituierte Benzochinolizine, Verfahren zur Herstellung und ihre Verwendung in Arzneimitteln.**

(57) Die Erfindung betrifft neue substituierte Benzochinolizine der allgemeinen Formel

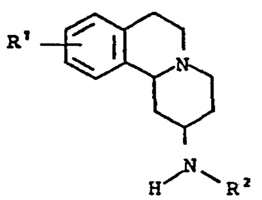

(I)

in welcher die Substituenten $R^1$ und $R^2$ die in der Beschreibung angegebene Bedeutung haben, Verfahren zu ihrer Herstellung sowie ihre Verwendung in Arzneimitteln, insbesondere in kreislaufbeeinflussenden Arzneimitteln.

0203441

86106401.2

BAYER AKTIENGESELLSCHAFT
Konzernverwaltung RP
Patentabteilung

5090 Leverkusen, Bayerwerk

KS/by-c
Ia (Pha)

Substituierte Benzochinolizine, Verfahren zur Herstellung und ihre Verwendung in Arzneimitteln

Die Erfindung betrifft neue substituierte Benzochinolizine, Verfahren zu ihrer Herstellung sowie ihre Verwendung in Arzneimitteln, insbesondere in kreislaufbeeinflussenden Arzneimitteln.

Es ist bekannt, daß in 8, 9, 10 oder 11-Stellung unsubstituierte 2-Anilino-hexahydrobenzo[a]chinolizine sowie 9,10-dimethoxysubstituierte 2-Anilino-hexahydrobenzo[a]-chinolizine blutdrucksenkend wirken (vgl. US 3.995.041; US 4.230.710).

Die vorliegende Erfindung betrifft Benzochinolizine der allgemeinen Formel (I)

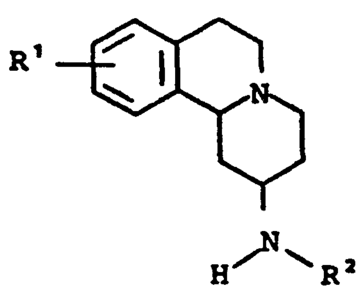

(I)

in welcher

$R^1$ - für 8-Halogen,

- für 9-Halogen oder
  für 9-Alkyl mit bis zu 8 C-Atomen, oder
  für 9-Halogenalkyl oder 9-Halogenalkoxy mit jeweils bis zu 8 C-Atomen und ein oder mehreren Fluor und/oder Chlor steht, oder
- für 9-Ethoxy steht, oder
- für 10-Halogen,
  für 8,9-Dialkyl mit bis zu 8 C-Atomen
  für 8,9-Dialkoxy mit bis zu 8 C-Atomen,
  für 8,9-Dialkylthio mit bis zu 8 C-Atomen,
  für 8,9-Bis-halogenalkyl mit bis zu 8 C-Atomen und ein oder mehreren Fluor und/oder Chlor steht, oder
- für 9,10-Dioxyalkylen mit bis zu 3 C-Atomen in der Alkylenkette steht, oder
- für 8,11-Dialkyl mit bis zu 8 C-Atomen,
  für 8,11-Dialkoxy mit bis zu 8 C-Atomen,
  für 8,11-Dialkylthio mit bis zu 8 C-Atomen,
  oder für 8,9-Bis-halogenalkyl mit bis zu 8 C-Atomen und ein oder mehreren Fluor und/oder Chlor steht,

und

$R^2$ - für einen Phenylrest steht, der bis zu fünffach, gleich oder verschieden substituiert ist durch Halogen, Cyano, Nitro,
durch Alkyl, Alkoxy, Alkylthio mit jeweils bis zu 8 C-Atomen, oder durch Halogenalkyl mit bis zu 8 C-Atomen und ein oder mehreren Fluor und/oder Chlor,

durch Amidino, Guanidino oder Formyl,
durch $SO_3H$, $SO_2$-Alkyl mit bis zu 6 C-Atomen,
-$SO_2$-Phenyl, -$SO_2$-Tolyl,
durch Acyl mit bis zu 8 C-Atomen, Alkoxycarbonyl mit bis zu 8 C-Atomen, oder
durch -$SO_2NR^3R^4$, -$CONR^3R^4$ oder -$NR^5R^6$,

wobei

$R^3$,$R^4$ - gleich oder verschieden sind und
- für Wasserstoff,
- für Alkyl mit bis zu 8 C-Atomen,
- für Phenyl, Benzyl oder Acetyl stehen,

$R^5$ - für Wasserstoff oder
- für Alkyl mit bis zu 8 C-Atomen steht,

und

$R^6$ - für Wasserstoff,
- für Acyl mit bis zu 8 C-Atomen,
- für Alkyl mit bis zu 8 C-Atomen,
- für Aryl mit 6 oder 10 C-Atomen,
- für $SO_2$-Alkyl mit bis zu 8 C-Atomen,
- für $SO_2$-Phenyl, $SO_2$-Tolyl oder -$SO_2NH_2$,
- für Alkoxycarbonyl mit bis zu 8 C-Atomen oder
- für -$CONR^3R^4$ steht, wobei $R^3$, $R^4$ die oben bereits angegebene Bedeutung haben,

und deren pharmakologisch annehmbaren nicht-toxischen Säureadditionsprodukte.

Die erfindungsgemäßen Stoffe sind stark blutdrucksenkend und können somit als Wirkstoffe in Arzneimitteln eingesetzt werden.

Bevorzugt sind solche Verbindungen der allgemeinen Formel (I), in welchen

$R^1$
- für 8-Fluor, 8-Chlor oder 8-Brom steht, oder
- für 9-Fluor, 9-Chlor oder 9-Brom steht, oder
- für 9-Alkyl mit bis zu 6 C-Atomen steht, oder
- für 9-Halogenalkyl oder 9-Halogenalkoxy mit jeweils bis zu 6 C-Atomen und ein oder mehreren Fluor steht, oder
- für 9-Ethoxy steht, oder
- für 10-Fluor, 10-Chlor, 10-Brom steht,
- für 8,9-Dialkyl mit bis zu 6 C-Atomen, für 8,9-Dialkoxy mit bis zu 6 C-Atomen, für 8,9-Dialkylthio mit bis zu 6 C-Atomen, für 8,9-Bis-halogenalkyl mit bis zu 6 C-Atomen und ein oder mehreren Fluor steht, oder
- für 9,10-Dioxyalkylen mit bis zu 2 C-Atomen in der Alkylenkette steht oder
- für 8,11-Dialkyl mit bis zu 6 C-Atomen, für 8,11-Dialkoxy mit bis zu 6 C-Atomen, für 8,11-Dialkylthio mit bis zu 6 C-Atomen, oder für 8,11-Bis-halogenalkyl mit bis zu 6 C-Atomen und ein oder mehreren Fluor steht

und

$R^2$ - für einen Phenylrest steht, der bis zu vierfach gleich oder verschieden substituiert ist durch Fluor, Chlor, Brom, Cyano, Nitro, durch Alkyl, Alkoxy, Alkylthio mit jeweils bis zu 6 C-Atomen, oder durch Halogenalkyl mit bis zu 6 C-Atomen und ein oder mehreren Fluor, durch Amidino , Guanidino, durch $SO_3H$, $SO_2$-Alkyl mit bis zu 4 C-Atomen, $SO_2$-Phenyl, -$SO_2$-Tolyl, durch Benzoyl oder Acetyl, durch Alkoxycarbonyl mit bis zu 6 C-Atomen, oder durch -$SO_2NR^3R^4$, $CONR^3R^4$ oder $NR^5R^6$,

wobei

$R^3$,$R^4$ - gleich oder verschieden sind und
- für Wasserstoff,
- für Alkyl mit bis zu 6 C-Atomen,
- für Phenyl oder Benzyl stehen,

$R^5$ - für Wasserstoff oder
- für Alkyl mit bis zu 6 C-Atomen steht,

und

$R^6$ - für Wasserstoff,
- für CO-Alkyl mit bis zu 6 C-Atomen oder Benzoyl,
- für Alkyl mit bis zu 6 C-Atomen,
- für Phenyl,
- für $SO_2$-Alkyl mit bis zu 5 C-Atomen,
- für $SO_2$-Phenyl oder $SO_2$-Tolyl,
- für Alkoxycarbonyl mit bis zu 6 C-Atomen, oder
- für $CONR^3R^4$ steht, wobei $R^3$, $R^4$ die oben angegebene Bedeutung haben

und deren pharmakologisch annehmbare nicht-toxische Säureadditionsprodukte.

Besonders bevorzugt sind diejenigen Verbindungen der allgemeinen Formel (I), in welchen

$R^1$ - für 8-Fluor oder 8-Chlor steht, oder
- für 9-Fluor oder 9-Chlor steht, oder
- für 9-Alkyl mit bis zu 3 C-Atomen steht, oder
- für 9-Trifluormethyl, 9-Trifluormethoxy steht, oder
- für 9-Ethoxy steht, oder
- für 10-Fluor oder 10-Chlor steht,
- für 8,9-Dialkyl mit bis zu 3 C-Atomen,
- für 8,9-Dialkoxy mit bis zu 3 C-Atomen,
- für 8,9-Dialkylthio mit bis zu 3 C-Atomen
- für 8,9-Bis-trifluormethyl steht, oder
- für 9,10-Dioxyalkylen mit bis zu 2 C-Atomen in der Alkylenkette steht, oder
- für 8,11-Dialkyl mit bis zu 3 C-Atomen,
für 8,11-Dialkoxy mit bis zu 3 C-Atomen,
für 8,11-Dialkylthio mit bis zu 3 C-Atomen, oder
für 8,11-Bis-trifluormethyl steht

und

$R^2$ - für einen Phenylrest steht, der bis zu dreifach gleich oder verschieden substituiert ist durch Fluor, Chlor, Cyano, Nitro, durch Alkyl, Alkoxy, Alkylthio mit jeweils bis zu 4 C-Atomen,
durch Trifluormethyl,
durch Amidino,

durch $SO_2$-Alkyl mit bis zu 3 C-Atomen, $SO_2$-Phenyl, $SO_2$-Tolyl,
durch Benzoyl oder Acetyl,
durch Alkoxycarbonyl mit bis zu 4 C-Atomen,
durch $SO_2NR^3R^4$, $CONR^3R^4$ oder $NR^5R^6$,

wobei

$R^3$, $R^4$ - gleich oder verschieden sind und
- für Wasserstoff,
- für Alkyl mit bis zu 4 C-Atomen,
- für Benzyl oder Phenyl stehen,

$R^5$ - für Wasserstoff oder
- für Alkyl mit bis zu 4 C-Atomen steht

und

$R^6$ - für Wasserstoff,
- für CO-Alkyl mit bis zu 4 C-Atomen, oder Benzoyl,
- für Phenyl,
- für $SO_2$-Alkyl mit bis zu 3 C-Atomen,
- für $SO_2$-Phenyl oder $SO_2$-Tolyl,
- für Alkoxycarbonyl mit bis zu 4 C-Atomen, oder
- für $CONR^3R^4$ steht, wobei $R^3$, $R^4$ die oben angegebene Bedeutung haben,

und deren pharmakologisch annehmbare nicht-toxische Säureadditionsprodukte.

Ganz besonders bevorzugt sind Verbindungen der allgemeinen Formel (I), in welchen

$R^1$ - für 8-Chlor,
- für 9-Fluor, 9-Chlor, 9-Methyl, 9-Trifluormethoxy oder 9-Ethoxy,
- für 10-Chlor, oder
- für 8,9-Dimethyl oder 8,9-Dimethoxy steht, oder
- für 9,10-Dioxymethylen steht, oder
- für 8,11-Dimethyl oder 8,11-Dimethoxy steht

und

$R^2$ - für einen Phenylrest steht, der bis zu dreifach gleich oder verschieden substituiert ist durch Fluor, Chlor, Brom, Cyano, Nitro, durch Alkyl, Alkoxy, Alkylthio mit jeweils bis zu 6 C-Atomen, oder durch Halogenalkyl mit bis zu 6 C-Atomen und ein oder mehreren Fluor, durch Amidino, Guanidino, durch $SO_3H$, $SO_2$-Alkyl mit bis zu 4 C-Atomen, $SO_2$-Phenyl, -$SO_2$-Tolyl, durch Benzoyl oder Acetyl, durch Alkoxycarbonyl mit bis zu 6 C-Atomen, oder durch $-SO_2NR^3R^4$, $CONR^3R^4$ oder $NR^5R^6$,

wobei

$R^3$,$R^4$ - gleich oder verschieden sind und
- für Wasserstoff,
- für Alkyl mit bis zu 6 C-Atomen
- für Phenyl oder Benzyl stehen,

$R^5$ - für Wasserstoff oder
- für Alkyl mit bis zu 6 C-Atomen steht

und

$R^6$ - für Wasserstoff,
- für CO-Alkyl mit bis zu 6 C-Atomen oder Benzoyl,
- für Alkyl mit bis zu 6 C-Atomen, oder
- für Phenyl oder
- für $SO_2$-Alkyl mit bis zu 5 C-Atomen,
- für $SO_2$-Phenyl oder $SO_2$-Tolyl,
- für Alkoxycarbonyl mit bis zu 6 C-Atomen, oder
- für $CONR^3R^4$ steht, wobei $R^3$, $R^4$ die oben angegebene Bedeutung haben,

und deren pharmakologisch annehmbare nicht-toxische Säureadditionsprodukte.

Die Erfindung betrifft Verbindungen der Formel (I) sowohl in Form ihrer freien Basen als auch in Form ihrer Säureadditionsprodukte. Bevorzugt sind die Säureadditionsprodukte von Verbindungen der Formel (I) an anorganische oder organische Säuren. Anorganische Säuren können die üblichen Säuren wie beispielsweise Chlorwasserstoff, Bromwasserstoff, Schwefelsäure, Phosphorsäure oder Perchlorsäure sein; organische Säuren können die üblichen Säuren wie beispielsweise Essigsäure, Oxalsäure, Maleinsäure, Fumarsäure, Milchsäure, Zitronensäure, Äpfelsäure oder Weinsäure sein, gegebenenfalls in D-, L-, Meso- oder racemischer Form.

Außerdem wurde gefunden, daß man die Verbindungen der allgemeinen Formel (I) erhält, wenn man

in einem ersten Schritt die Ketone der allgemeinen Formel (II)

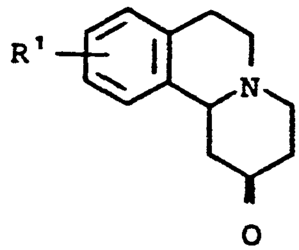

(II),

in welcher

$R^1$ die oben angegebene Bedeutung hat,

mit Aminen der allgemeinen Formel (III)

$$H_2N-R^2 \quad (III),$$

in welcher

$R^2$ die angegebene Bedeutung hat,

in einem inerten organischen Lösungsmittel gegebenenfalls in Gegenwart eines Katalysators umsetzt, die so erhaltenen Schiffschen Basen in einem zweiten Schritt in inerten organischen Lösungsmitteln in an sich bekannter Weise hydriert und gegebenenfalls anschließend die Verbindungen der Formel (I) mit Säuren in deren pharmakologisch annehmbare nicht-toxische Salze überführt.

Die erfindungsgemäßen Verbindungen besitzen zwei asymmetrische Kohlenstoffatome und können daher in verschiedenen stereochemischen Formen existieren. Außerdem können sie als cis- oder trans-Isomere vorliegen [vgl. J. Gootjes, A.M. DeRoos, W.T. Nauter, Recl.Trav.Chim. Pays-Bas 85, 491 (1966)]. Die Erfindung betrifft sowohl die einzelnen Isomeren (A bzw. B) als auch deren Mischungen.

Die erfindungsgemäßen Verbindungen senken den Blutdruck. Überraschenderweise haben sie eine erschlaffende Wirkung auf das venöse System und können somit als Arzneimittel eingesetzt werden. Sie stellen damit eine Bereicherung der Pharmazie dar.

Verwendet man als Ausgangsmaterial 9-Chlor-1,3,4,6,7,11-b-hexahydro-2-oxo-2H-benzo[a]chinolizin und 3,4,5-Trimethoxyanilin, so läßt sich die Synthese der erfindungsgemäßen Verbindungen durch folgendes Schema verdeutlichen:

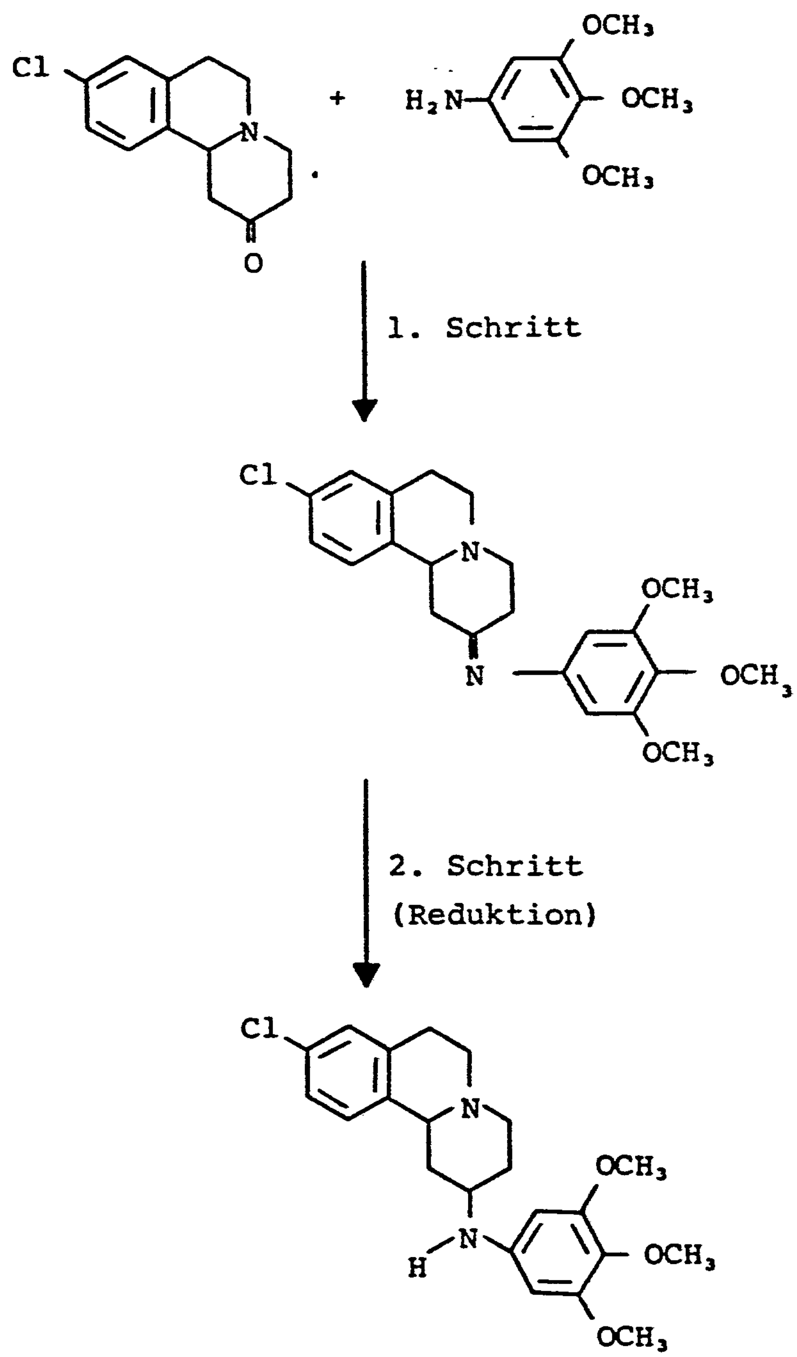
1. Schritt
2. Schritt
(Reduktion)

Die Ausgangsstoffe der allgemeinen Formel (II) sind bekannt oder können nach bekannten Methoden hergestellt werden [vgl. D. Beke, C. Szantay, Chem. Ber. 95, 2132 (1962)].

Als Verdünnungsmittel für den ersten Schritt kommen alle inerten organischen Lösungsmittel in Frage. Bevorzugt sind aromatische Kohlenwasserstoffe wie Benzol, Toluol oder Xylol, Dimethylformamid oder deren Gemische. Als Katalysator werden im allgemeinen Säuren verwendet. Bevorzugt sind Säuren wie z.B. Salzsäure, Schwefelsäure oder organische Sulfonsäuren wie z.B. Toluolsulfonsäure, Benzolsulfonsäure, Methan- oder Ethansulfonsäure. Ganz besonders bevorzugt wird bei der Umsetzung Toluolsulfonsäure eingesetzt.

Die Reaktionstemperaturen können in einem größeren Bereich variiert werden. Im allgemeinen arbeitet man zwischen 60°C und 200°C, bevorzugt bei der Siedetemperatur des verwendeten Lösungsmittels.

Die Umsetzung kann bei normalem Druck aber auch bei erhöhtem Druck durchgeführt werden. Im allgemeinen arbeitet man bei Normaldruck.

Bei der Durchführung der Reaktion werden die Ausgangsstoffe in einem molaren Verhältnis von Keton II zu Amin III von 1:1 bis 1:2 eingesetzt. Bevorzugt werden molare Mengen der Reaktanden eingesetzt.

Die Hydrierung der Schiffschen Base erfolgt nach den üblichen Methoden entweder in inerten organischen Lösungsmitteln wie Alkohole, Ether oder Chlorkohlenwasserstoffen mit Katalysatoren wie Platin, Palladium oder Palladium auf Tierkohle, oder mit Hydriden in inerten organischen Lösungsmitteln.

Bevorzugt wird die Hydrierung mit Hydriden wie $LiAlH_4$ oder $NaBH_4$, in inerten organischen Lösungsmitteln, bevorzugt in Alkoholen wie Methanol, Ethanol oder Propanol, in Ethern wie Diethylether, Dioxan oder Tetrahydrofuran durchgeführt. Ganz besonders bevorzugt wird im zweiten Schritt mit Natriumborhydrid in Alkoholen wie Methanol, Ethanol oder Propanol hydriert.

Die erfindungsgemäßen Wirkstoffe weisen eine blutdrucksenkende Wirkung auf. Durch Erweiterung der Venenkapazität wird der venöse Rückstrom zum Herzen, die Füllung der Herzventrikel und damit die Pumpleistung des Herzens vermindert. Aufgrund dieser Eigenschaften können die erfindungsgemäßen Verbindungen zur Behandlung von Herz-Kreislauf-Erkrankungen wie Angina pectoris, Herzversagen oder Bluthochdruck eingesetzt werden.

Die Wirkung der erfindungsgemäßen Stoffe auf die Venenkapazität wird an der narkotisierten Katze mittels eines Plethysmographen gemessen. Als Vergleich wird in jedem Versuch als Standardsubstanz Natrium-Nitroprussid untersucht.

Tabelle 1 zeigt beispielhaft die pharmakologische Wirkung der erfindungsgemäßen Wirkstoffe an der ketaminanästhesierten Katze.

Tabelle 1

| Beispiel-Nummer | Dosis mg/kg | Venenkapazität | art.Mitteldruck |
|---|---|---|---|
| 3 | 0,03 i.v. | + 15 % | - 17 % |
| | 0,1 i.v. | + 37 % | - 20 % |
| | 0,3 i.v. | + 31 % | - 25 % |
| | 1,0 i.v. | + 30 % | - 26 % |
| 21 | 0,03 i.v. | + 11 % | - 14 % |
| | 0,1 i.v. | + 34 % | - 22 % |
| | 0,3 i.v. | + 30 % | - 24 % |
| | 1,0 i.v. | + 36 % | - 29 % |
| 26 | 0,1 i.v. | + 29 % | ø |
| | 1,0 i.v. | + 47 % | - 12 % |
| | 3,1 i.v. | + 45 % | - 31 % |
| 38 | 0,3 i.v. | + 26 % | - 16 % |
| | 1,0 i.v. | + 38 % | - 23 % |
| | 3,1 i.v. | + 32 % | - 44 % |
| 42 | 1,0 i.v. | + 9 % | - 12 % |
| | 3,1 i.v. | + 31 % | - 29 % |
| | 10,0 i.v. | + 28 % | - 47 % |

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstreckung der Wirkstoffe mit Lösungsmittel und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin, Sulfitablaugen,

Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und/oder dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendung gedacht sind, können die Wirkstoffe außer den obengenannten Hilfsstoffen mit verschiedenen Geschmackaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

0203441

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierfür auch die obigen Ausführungen.

## Herstellungsbeispiele

Allgemeine Arbeitsvorschrift zur Herstellung von substituierten 2-Anilino-1,3,4,6,7,11b-hexahydro-2H-benzo[a]-chinolizinen (Formel I)

Eine Mischung aus substituierten 1,3,4,6,7,11b-Hexahydro-2-oxo-2H-benzo[a]chinolizin (Formel II) (0,04 Mol), substituierten Anilin (Formel III) (0,04 Mol), einer katalytischen Menge p-Toluolsulfonsäure, 25 ml Dimethylformamid und 150 ml Benzol werden 18 Stunden rückflußbehandelt und das gebildete Wasser durch einen Wasserabscheider abgetrennt. Das Lösungsmittel wird im Vakuum eingeengt, wobei ein Öl zurückbleibt, das in 150 ml Methanol gelöst wird. Die Lösung wird auf einem Eisbad gekühlt und dazu wird portionsweise Natriumborhydrid (7 g) gegeben. Die Mischung wird in der Kälte eine Stunde gerührt und dann 1,5 Stunden rückflußbehandelt. Das Lösungsmittel wird im Vakuum eingeengt, zum Rückstand wird Wasser gegeben und die Mischung mit Chloroform mehrmals extrahiert. Nach dem Trocknen über Magnesiumsulfat verbleibt aus der organischen Phase ein Öl, das an einer Kieselgelsäule (Laufmittel: Chloroform/Methanol 3:1, oder Toluol/Ethanol/Triethylamin 10:3:1) aufgetrennt wird. Die Fraktionen, die mit Schlittlers Reagenz eine positive Reaktion ergeben, werden isoliert. Der zuerst eluierte Teil wird als Isomer A bezeichnet, der zweite Teil als Isomer B. Die reinen Isomeren oder die Isomerengemische werden durch ethanolische, etherische oder isopropanolische Salzsäure in die Hydrochloride überführt. Tartrate werden mit einer Lösung mit Weinsäure in Isopropanol hergestellt.

Die ausfallenden Salze werden abfiltriert und noch feucht im Hochvakuum von restlichen Lösungsmittelanteilen bereitet.

Die in den folgenden Tabellen aufgeführten Beispiele wurden nach dem beschriebenen Verfahren hergestellt:

Tabelle 2

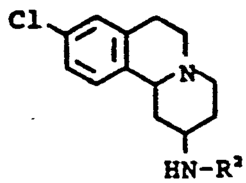

| Bsp.-Nr. | $R^2$ | Isomeres | Salz mit | Schmelzpunkt [°C] |
|---|---|---|---|---|
| 1 | 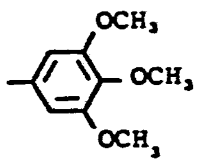 | A | L(+)-Weinsäure | 95 (Zers.) |
| 2 | " | B | " | 85 |
| 3 | 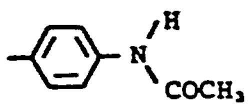 | A | " | 70 |
| 4 | " | B | " | 65 |
| 5 | " | A+B | " | 95 |
| 6 | 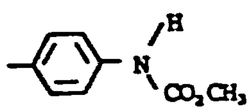 | A | meso-Weinsäure | 115 |
| 7 | 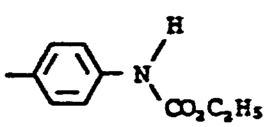 | A | HCl | |
| 8 | 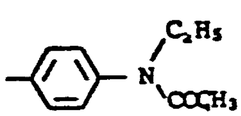 | A | L(+)-Weinsäure | 70 |
| 9 | " | B | " | |

Tabelle 2 (Fortsetzung)

| Bsp.-Nr. | $R^2$ | Isomeres | Salz mit | Schmelzpunkt [°C] |
|---|---|---|---|---|
| 10 | 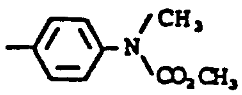 | A+B | L(+)-Weinsäure | |
| 11 | 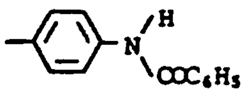 | A | " | 110 |
| 12 | " | B | " | 110-120 |
| 13 | " | A | HCl | 130 (Zers.) |
| 14 | " | B | HCl | 135 (Zers.) |

Tabelle 3

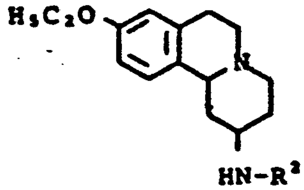

| Bsp.-Nr. | $R^2$ | Isomeres | Salz mit | Schmelzpunkt [°C] |
|---|---|---|---|---|
| 15 | 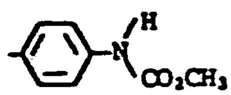 | A | HCl | 198-200 |
| 16 | " | B | " | 200 (Zers.) |
| 17 | 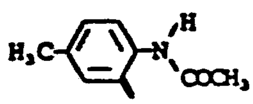 | A | " | 171-3 (Zers.) |
| 18 | " | B | " | 165-7 (Zers.) |
| 19 | 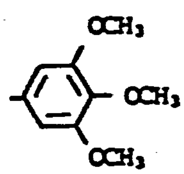 | A | " | 175-7 (Zers.) |
| 20 | " | B | " | 170-2 (Zers.) |
| 21 | 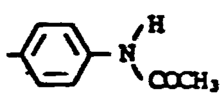 | A | " | 168-70 |
| 22 | 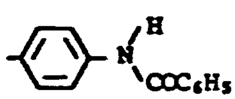 | A | " | 178-80 |

Tabelle 3 (Fortsetzung)

| Bsp.-Nr. | $R^2$ | Isomeres | Salz mit | Schmelzpunkt [°C] |
|---|---|---|---|---|
| 23 | 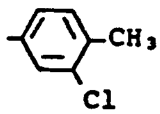 | A | " | 153-5 (Zers.) |
| 24 | 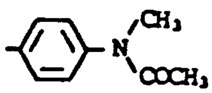 | A | " | 190-2 |
| 25 | 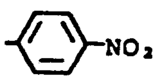 | A | " | 158-60 |
| 26 | 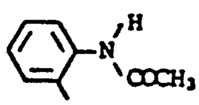 | A | " | 163-5 (Zers.) |

Tabelle 4

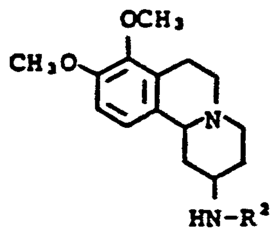

| Bsp.-Nr. | $R^2$ | Isomers | Salz mit | Schmelzpunkt [°C] |
|---|---|---|---|---|
| 27 | 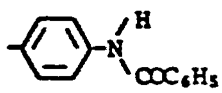 | A | HCl | 250-2 |
| 28 | " | B | " | >260 |
| 29 | 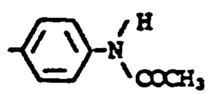 | A | " | >260 |
| 30 | " | B | " | >260 |
| 31 | 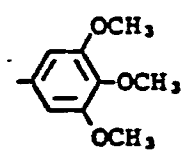 | A | " | 185-7 |
| 32 | " | B | " | 200-2 |
| 33 | 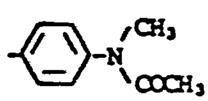 | B | " | 210 (Zers.) |

Tabelle 5

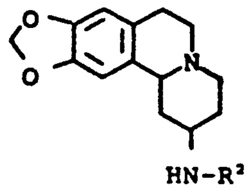

| Bsp.-Nr. | $R^2$ | Isomeres | Salz mit | Schmelzpunkt [°C] |
|---|---|---|---|---|
| 34 | 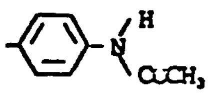 | A | HCl | 233-5 |
| 35 | " | B | " | 238-40 |
| 36 | 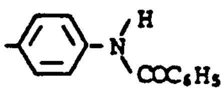 | A | " | 278-80 |
| 37 | " | B | " | 220-2 |
| 38 | 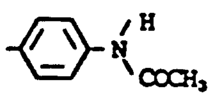 | A | " | 250-2 |
| 39 | " | B | " | 245-7 |
| 40 | 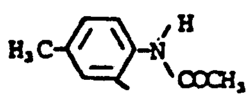 | A | " | 270-3 |

Tabelle 6

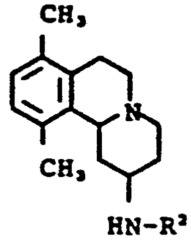

| Bsp.-Nr. | $R^2$ | Isomeres | Salz mit | Schmelzpunkt [°C] |
|---|---|---|---|---|
| 41 | 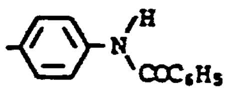 | A | HCl | 227-9 |
| 42 | " | B | " | 218-20 |
| 43 | 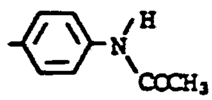 | A | " | 213-5 |
| 44 | 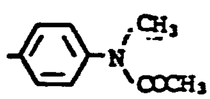 | A | " | 210-2 |
| 45 | " | B | " | 205-7 |

Patentansprüche

1. Benzochinolizine der allgemeinen Formel (I)

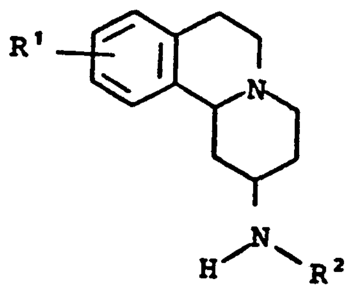

(I)

in welcher

$R^1$ - für-8 Halogen,

- für 9-Halogen oder für 9-Alkyl mit bis zu 8 C-Atomen, oder für 9-Halogenalkyl oder 9-Halogenalkoxy mit jeweils bis zu 8 C-Atomen und ein oder mehreren Fluor und/oder Chlor steht, oder
- für 9-Ethoxy steht, oder
- für 10-Halogen
- für 8,9-Dialkyl mit bis zu 8 C-Atomen für 8,9-Dialkoxy mit bis zu 8 C-Atomen, für 8,9-Dialkylthio mit bis zu 8 C-Atomen, für 8,9-Bis-halogenalkyl mit bis zu 8 C-Atomen und ein oder mehreren Fluor und/oder Chlor steht, oder
- für 9,10-Dioxyalkylen mit bis zu 3 C-Atomen in der Alkylenkette steht, oder
- für 8,11-Dialkyl mit bis zu 8 C-Atomen, für 8,11-Dialkoxy mit bis zu 8 C-Atomen, für 8,11-Dialkylthio mit bis zu 8 C-Atomen, oder für 8,9-Bis-halogenalkyl mit bis zu 8 C-Atomen und ein oder mehreren Fluor und/oder Chlor steht,

und

$R^2$ - für einen Phenylrest steht, der bis zu fünffach, gleich oder verschieden substituiert ist durch Halogen, Cyano, Nitro,
durch Alkyl, Alkoxy, Alkylthio mit jeweils bis zu 8 C-Atomen, oder durch Halogenalkyl mit bis zu 8 C-Atomen und ein oder mehreren Fluor und/oder Chlor,
durch Amidino, Guanidino oder Formyl,
durch $SO_3H$, $SO_2$-Alkyl mit bis zu 6 C-Atomen, $-SO_2$-Phenyl, $-SO_2$-Tolyl,
durch Acyl mit bis zu 8 C-Atomen, Alkoxycarbonyl mit bis zu 8 C-Atomen, oder
durch $-SO_2NR^3R^4$, $-CONR^3R^4$ oder $-NR^5R^6$,

wobei

$R^3$,$R^4$ - gleich oder verschieden sind und
- für Wasserstoff,
- für Alkyl mit bis zu 8 C-Atomen,
- für Phenyl, Benzyl oder Acetyl stehen,

$R^5$ - für Wasserstoff oder
- für Alkyl mit bis zu 8 C-Atomen steht,

und

$R^6$ - für Wasserstoff,
- für Acyl mit bis zu 8 C-Atomen,
- für Alkyl mit bis zu 8 C-Atomen,
- für Aryl mit 6 oder 10 C-Atomen,
- für $SO_2$-Alkyl mit bis zu 8 C-Atomen,
- für $SO_2$-Phenyl, $SO_2$-Tolyl oder $-SO_2NH_2$,
- für Alkoxycarbonyl mit bis zu 8 C-Atomen oder
- für $-CONR^3R^4$ steht, wobei $R^3$, $R^4$ die oben bereits angegebene Bedeutung haben,

und deren pharmakologisch annehmbaren nicht-toxischen Säureadditionsprodukte.

2. Verbindungen der allgemeinen Formel (I), in welcher

$R^1$ - für 8-Fluor, 8-Chlor oder 8-Brom steht, oder
- für 9-Fluor, 9-Chlor oder 9-Brom steht, oder
- für 9-Alkyl mit bis zu 6 C-Atomen steht, oder
- für 9-Halogenalkyl oder 9-Halogenalkoxy mit jeweils bis zu 6 C-Atomen und ein oder mehreren Fluor steht, oder
- für 9-Ethoxy steht, oder
- für 10-Fluor, 10-Chlor, 10-Brom steht,
- für 8,9-Dialkyl mit bis zu 6 C-Atomen, für 8,9-Dialkoxy mit bis zu 6 C-Atomen, für 8,9-Dialkylthio mit bis zu 6 C-Atomen, für 8,9-Bis-halogenalkyl mit bis zu 6 C-Atomen und ein oder mehreren Fluor steht, oder

- für 9,10-Dioxyalkylen mit bis zu 2 C-Atomen in der Alkylenkette steht oder
- für 8,11-Dialkyl mit bis zu 6 C-Atomen, für 8,11-Dialkoxy mit bis zu 6 C-Atomen, für 8,11-Dialkylthio mit bis zu 6 C-Atomen, oder für 8,11-Bis-halogenalkyl mit bis zu 6 C-Atomen und ein oder mehreren Fluor steht

und

$R^2$ - für einen Phenylrest steht, der bis zu vierfach gleich oder verschieden substituiert ist durch Fluor, Chlor, Brom, Cyano, Nitro, durch Alkyl, Alkoxy, Alkylthio mit jeweils bis zu 6 C-Atomen, oder durch Halogenalkyl mit bis zu 6 C-Atomen und ein oder mehreren Fluor, durch Amidin, Guanidin, durch $SO_3H$, $SO_2$-Alkyl mit bis zu 4 C-Atomen, $SO_2$-Phenyl, -$SO_2$-Tolyl, durch Benzoyl oder Acetyl, durch Alkoxycarbonyl mit bis zu 6 C-Atomen, oder durch $-SO_2NR^3R^4$, $CONR^3R^4$ oder $NR^5R^6$,

wobei

$R^3$,$R^4$ - gleich oder verschieden sind und
- für Wasserstoff,
- für Alkyl mit bis zu 6 C-Atomen,
- für Phenyl oder Benzyl stehen,

$R^5$ - für Wasserstoff oder
- für Alkyl mit bis zu 6 C-Atomen steht,

und

$R^6$ - für Wasserstoff,
- für CO-Alkyl mit bis zu 6 C-Atomen oder Benzoyl,
- für Alkyl mit bis zu 6 C-Atomen, oder
- für Phenyl oder
- für $SO_2$-Alkyl mit bis zu 5 C-Atomen,
- für $SO_2$-Phenyl oder $SO_2$-Tolyl,
- für Alkoxycarbonyl mit bis zu 6 C-Atomen, oder
- für $CONR^3R^4$ steht, wobei $R^3$, $R^4$ die oben angegebene Bedeutung haben.

3. Verbindungen der allgemeinen Formel (I), in welcher

$R^1$ - für 8-Fluor oder 8-Chlor steht, oder
- für 9-Fluor oder 9-Chlor steht, oder
- für 9-Alkyl mit bis zu 3 C-Atomen steht, oder
- für 9-Trifluormethyl, 9-Trifluormethoxy steht, oder
- für 9-Ethoxy steht, oder
- für 10-Fluor oder 10-Chlor steht,
- für 8,9-Dialkyl mit bis zu 3 C-Atomen,
- für 8,9-Dialkoxy mit bis zu 3 C-Atomen,
- für 8,9-Dialkylthio mit bis zu 3 C-Atomen
- für 8,9-Bis-trifluormethyl steht, oder
- für 9,10-Dioxyalkylen mit bis zu 2 C-Atomen in der Alkylenkette steht, oder

- für 8,11-Dialkyl mit bis zu 3 C-Atomen,
  für 8,11-Dialkoxy mit bis zu 3 C-Atomen,
  für 8,11-Dialkylthio mit bis zu 3 C-Atomen,
  oder
  für 8,11-Bis-trifluormethyl steht

und

$R^2$ - für einen Phenylrest steht, der bis zu dreifach gleich oder verschieden substituiert ist durch Fluor, Chlor, Cyano, Nitro, durch Alkyl, Alkoxy, Alkylthio mit jeweils bis zu 4 C-Atomen,
durch Trifluormethyl,
durch Amidino,
durch $SO_2$-Alkyl mit bis zu 3 C-Atomen,
$SO_2$-Phenyl, $SO_2$-Tolyl,
durch Benzoyl oder Acetyl,
durch Alkoxycarbonyl mit bis zu 4 C-Atomen,
durch $SO_2NR^3R^4$, $CONR^3R^4$ oder $NR^5R^6$,

wobei

$R^3$, $R^4$ - gleich oder verschieden sind und
- für Wasserstoff,
- für Alkyl mit bis zu 4 C-Atomen,
- für Benzyl oder Phenyl stehen,

$R^5$ - für Wasserstoff oder
- für Alkyl mit bis zu 4 C-Atomen steht

und

$R^6$ - für Wasserstoff,
- für CO-Alkyl mit bis zu 4 C-Atomen, oder Benzoyl,
- für Phenyl,
- für $SO_2$-Alkyl mit bis zu 3 C-Atomen,
- für $SO_2$-Phenyl oder $SO_2$-Tolyl,
- für Alkoxycarbonyl mit bis zu 4 C-Atomen, oder
- für $CONR^3R^4$ steht, wobei $R^3$, $R^4$ die oben angegebene Bedeutung haben.

4. Verbindungen der allgemeinen Formel (I), in welcher

$R^1$ - für 8-Chlor,
- für 9-Fluor, 9-Chlor, 9-Methyl, 9-Trifluormethoxy oder 9-Ethoxy,
- für 10-Chlor, oder
- für 8,9-Dimethyl oder 8,9-Dimethoxy steht, oder
- für 9,10-Dioxymethylen steht, oder
- für 8,11-Dimethyl oder 8,11-Dimethoxy steht

und

$R^2$ - für einen Phenylrest steht, der bis zu dreifach gleich oder verschieden substituiert ist durch Fluor, Chlor, Brom, Cyano, Nitro, durch Alkyl, Alkoxy, Alkylthio mit jeweils bis zu 6 C-Atomen, oder durch Halogenalkyl mit bis zu 6 C-Atomen und ein oder mehreren Fluor, durch Amidino,

Guanidino, durch $SO_3H$, $SO_2$-Alkyl mit bis zu 4 C-Atomen, $SO_2$-Phenyl, -$SO_2$-Tolyl, durch Benzoyl oder Acetyl, durch Alkoxycarbonyl mit bis zu 6 C-Atomen, oder durch -$SO_2NR^3R^4$, $CONR^3R^4$ oder $NR^5R^6$,

wobei

$R^3$,$R^4$ - gleich oder verschieden sind und
- für Wasserstoff,
- für Alkyl mit bis zu 6 C-Atomen
- für Phenyl oder Benzyl stehen,

$R^5$ - für Wasserstoff oder
- für Alkyl mit bis zu 6 C-Atomen steht

und

$R^6$ - für Wasserstoff,
- für CO-Alkyl mit bis zu 6 C-Atomen oder Benzoyl,
- für Alkyl mit bis zu 6 C-Atomen, oder
- für Phenyl oder
- für $SO_2$-Alkyl mit bis zu 5 C-Atomen,
- für $SO_2$-Phenyl oder $SO_2$-Tolyl,
- für Alkoxycarbonyl mit bis zu 6 C-Atomen, oder
- für $CONR^3R^4$ steht, wobei $R^3$, $R^4$ die oben angegebene Bedeutung haben.

5. Verfahren zur Herstellung von Verbindungen der allgemeinen Formel (I)

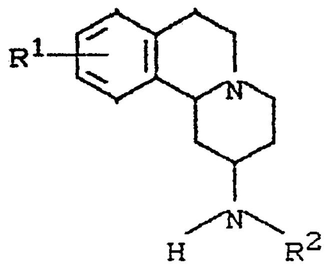

(I)

in welcher

$R^1$ - für-8 Halogen,
- für 9-Halogen oder für 9-Alkyl mit bis zu 8 C-Atomen, oder für 9-Halogenalkyl oder 9-Halogenalkoxy mit jeweils bis zu 8 C-Atomen und ein oder mehreren Fluor und/oder Chlor steht, oder
- für 9-Ethoxy steht, oder
- für 10-Halogen
- für 8,9-Dialkyl mit bis zu 8 C-Atomen für 8,9-Dialkoxy mit bis zu 8 C-Atomen, für 8,9-Dialkylthio mit bis zu 8 C-Atomen, für 8,9-Bis-halogenalkyl mit bis zu 8 C-Atomen und ein oder mehreren Fluor und/oder Chlor steht, oder
- für 9,10-Dioxyalkylen mit bis zu 3 C-Atomen in der Alkylenkette steht, oder
- für 8,11-Dialkyl mit bis zu 8 C-Atomen, für 8,11-Dialkoxy mit bis zu 8 C-Atomen, für 8,11-Dialkylthio mit bis zu 8 C-Atomen, oder für 8,9-Bis-halogenalkyl mit bis zu 8 C-Atomen und ein oder mehreren Fluor und/oder Chlor steht,

und

$R^2$ - für einen Phenylrest steht, der bis zu fünffach, gleich oder verschieden substituiert ist durch Halogen, Cyano, Nitro, durch Alkyl, Alkoxy, Alkylthio mit jeweils bis zu 8 C-Atomen, oder durch Halogenalkyl mit bis zu 8 C-Atomen und ein oder mehreren Fluor und/oder Chlor, durch Amidino, Guanidino oder Formyl, durch $SO_3H$, $SO_2$-Alkyl mit bis zu 6 C-Atomen, $-SO_2$-Phenyl, $-SO_2$-Tolyl, durch Acyl mit bis zu 8 C-Atomen, Alkoxycarbonyl mit bis zu 8 C-Atomen, oder durch $-SO_2NR^3R^4$, $-CONR^3R^4$ oder $-NR^5R^6$,

wobei

$R^3$,$R^4$ - gleich oder verschieden sind und
- für Wasserstoff,
- für Alkyl mit bis zu 8 C-Atomen,
- für Phenyl, Benzyl oder Acetyl steht,

$R^5$ - für Wasserstoff oder
- für Alkyl mit bis zu 8 C-Atomen steht,

und

$R^6$ - für Wasserstoff,
- für Acyl mit bis zu 8 C-Atomen,

- für Alkyl mit bis zu 8 C-Atomen,
- für Aryl mit 6 oder 10 C-Atomen,
- für $SO_2$-Alkyl mit bis zu 8 C-Atomen,
- für $SO_2$-Phenyl, $SO_2$-Tolyl oder $-SO_2NH_2$,
- für Alkoxycarbonyl mit bis zu 8 C-Atomen oder
- für $-CONR^3R^4$ steht, wobei $R^3$, $R^4$ die oben bereits angegebene Bedeutung haben,

dadurch gekennzeichnet, daß man in einem ersten Schritt die Ketone der allgemeinen Formel (II)

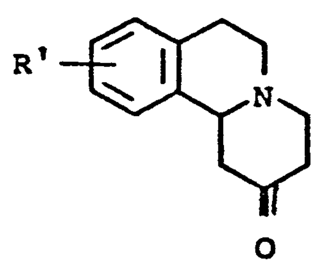

(II)

in welcher

$R^1$ die oben angegebene Bedeutung hat,

mit Aminen der allgemeinen Formel (III)

$$H_2N-R^2 \qquad (III)$$

in welcher

$R^2$ die oben angegebene Bedeutung hat

in einem inerten organischen Lösungsmittel gegebenenfalls in Gegenwart eines Katalysators umsetzt, die so erhaltenen Schiffschen Basen in einem zweiten Schritt in inerten organischen Lösungsmitteln in an sich bekannter Weise hydriert und gegebenenfalls anschließend die Verbindungen der Formel (I) mit Säuren in deren pharmakologisch annehmbare nicht-toxische Salze überführt.

6. Verbindungen der allgemeinen Formel (I) gemäß Anspruch 1 zur Verwendung bei der Bekämpfung von Kreislauferkrankungen.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man mindestens eine Verbindung der allgemeinen Formel (I) gemäß Anspruch 1 gegebenenfalls unter Verwendung weiterer Hilfs- und Trägerstoffe in eine geeignete Applikationsform überführt.

9. Verwendung von Verbindungen der allgemeinen Formel (I) gemäß Anspruch 1 bei der Herstellung von Arzneimitteln.

10. Verwendung von Verbindungen der allgemeinen Formel (I) gemäß Anspruch 1 bei der Herstellung von Kreislaufmitteln.